# EUROPEAN PATENT APPLICATION

(11) **EP 1 617 215 A1**
(43) Date of publication of application: **18.01.2006**
(21) Application number: 04728431.0
(22) Date of filing: 20.04.2004
(51) Int. Cl.: G01N 33/50, G01N 21/64

(54) **METHOD OF EVALUATING OXIDIZED PROTEIN IN HORNY CELL LAYER**

(30) Priority: 21.04.2003 JP 2003116157
(71) Applicant: SHISEIDO COMPANY LIMITED, Chuo-ku, Tokyo 104-8010 (JP)
(72) Inventor: FUJITA, Hiroshi, c/o SHISEIDO RESEARCH CENTER, Yokohama-shi, Kanagawa 24-8558 (JP); HIRAO, Tetsuji, c/o SHISEIDO RESEARCH CENTER, Yokohama-shi, Kanagawa 224-8558 (JP)
(74) Representative: Santarelli
(86) International application number: PCT/JP2004/005620
(87) International publication number: WO 2004/095022

(57) **Abstract**

The invention provides a method for two-dimensional evaluation of the nature of horny layer oxidized protein on a horny layer, the method comprising specific fluorescent labeling of the carbonyl groups of the oxidized protein in a horny layer specimen taken from skin, and detection of the fluorescence for evaluation, as well as a kit used to carry out the method and a method for screening of agents which inhibit increase in oxidized protein. The invention further provides a method for detecting the oxidized form of a cornified envelope consisting of the water-insoluble substances in a skin-derived horny layer specimen, the method comprising specific fluorescent labeling of the carbonyl groups in a cornified envelope in the oxidized form and detection of the fluorescence thereof for evaluation.

## Description

### Technical Field

The present invention relates to a method for two-dimensional evaluation of the attributes of horny layer oxidized protein in the skin's horny layer, characterized by specific fluorescent labeling of the oxidized protein, as well as a kit used for carrying out the method. The invention further provides a method for screening of agents which inhibit increase in oxidized protein caused by oxidizing agents such as sodium hypochlorite. The invention also relates to a method for detecting the oxidized form of the cornified envelope, a water-insoluble substance, in a skin-derived horny layer specimen.

### Background Art

Accurately assessing skin quality (or skin condition) is essential for proper skin care aimed at maintaining healthy skin. Thus, for skin care which is based on the use of cosmetics, it has become common to evaluate cosmetic user skin quality by the practice of interviewing with beauty specialists, for example. Various measuring devices are also used for objective evaluation of skin quality, whereby skin condition or function is evaluated based on observed or measured parameters.

A great amount of research has been conducted in recent years on horny layer oxidized protein in the context of intrinsic aging and photoaging of skin. Oxidized protein is a protein to which carbonyl groups had been introduced as a result of oxidation, and generally it includes a product resulting from direct oxidation of the NH₂ groups of amino acid residues such as Lys, Arg and Pro in a protein into carbonyl groups, and also a product resulting from a protein to which highly reactive aldehydes had been bind, wherein the aldehydes are formed from oxidation of lipids into lipid peroxides, followed by further decomposition. The relationship between oxidized protein and aging has been abundantly researched, and it has been found to be elevated in aging (brain, liver and fibroblasts), Alzheimer's disease and Werner's syndrome.

In skin, it is believed that oxidation of sebum on the skin surface by free radicals and the resulting lipid peroxides initiate oxidation of proteins. Once lipid peroxides are produced, oxidation proceeds in a chain reaction, not only irritating the skin surface but also damaging cells deep in the horny layer. The oxidation of sebum and skin proteins is thought to significantly reduce the function of skin for moisture retention, firmness and lightness. Consequently, evaluation of the attributes of oxidized proteins in the epidermis, such as their amounts and distribution, is considered to be extremely important for obtaining an accurate assessment of skin quality or skin condition, and for determining strategies and selecting cosmetics for methods of skin care.

As mentioned above, much research has been carried out in the study of horny layer oxidized protein. For example, in J.J. Thiele et al., FEBS Letter 1998, Feb. 6, 422(3), 403-406 there is described a method for detection of horny layer oxidized protein. This article discloses a method involving a tape stripping procedure in which adhesive tape is attached to the skin surface layer and then peeled off, to obtain tape with the horny layer adhering thereto ("tape-stripped horny layer"), and the oxidized protein is detected by ELISA. According to Thiele et al., ultraviolet irradiation of the tape horny layer resulted in an increase in oxidized protein, while the amount of oxidized protein was greater in the horny layer of light-exposed areas such as the face, as opposed to the horny layer of non-exposed areas such as the upper arms.

Also, J.J. Thiele et al., J. Invest. Dermatol. 1999, Sep, 113(3), 335-359 describes detection of oxidized protein by extraction of the protein from a tape horny layer, DNPH-labeling of the soluble components, SDS-PAGE and the use of anti-DNP antibody for Western blotting. It is reported in this article that oxidized protein was more abundant in the upper layer than in the middle or lower layers of the horny layer.

C.S. Sander et al., J. Invest. Dermatol. 2002, Apr, 118(4), 618-625 describes detection of oxidized protein by labeling human skin tissue thin sections with DNPH and staining with anti-DNP antibody. This article reports that oxidized protein levels are increased mainly in the dermis of photoaged skin, that horny layer oxidized protein levels increase with chronic ultraviolet exposure, and that the proportion of increase rises in a manner dependent on the ultraviolet exposure dose.

Thus, the plethora of research on horny layer oxidized protein suggests a correlation between intrinsic aging of skin and photoaging induced by ultraviolet irradiation. Evaluating the attributes of oxidized protein in skin would therefore be expected to provide useful information for selection of proper skin care methods, treatment methods, cosmetics and pharmaceuticals aimed at preventing or ameliorating skin aging.

Detection of oxidized protein according to the prior art has in all cases involved the laborious and time consuming procedure of taking a horny layer specimen, extracting the protein, using DNPH on the protein extract for labeling of the protein with DNP, separating by SDS-PAGE, transferring to a nitrocellulose membrane, using anti-DNP antibody and then binding peroxidase-labeled secondary antibody, and finally coloring with a chemiluminescent reagent (ECL substrate). In addition, the information obtained thereby is limited to amounts of oxidized protein levels, and absolutely no detailed two-dimensional information is obtained with regard to distribution and manifestation in the skin. Consequently, despite indications that detection of oxidized protein is useful for evaluation of skin quality, it has not been widely implemented because of the complicated procedure and the limited information obtained, as well as for other reasons. However, with simplification and a more detailed information content, it is believed that such detection could constitute an important means to aid in providing counseling services that offer advice for appropriate skin care methods, as has become common in the cosmetic field in recent years.

### Disclosure of the Invention

The present inventors focused on the goal of easily obtaining two-dimensional information in regard to the nature of oxidized protein found in the horny layer of skin, and utilizing such information on oxidized protein particularly for counseling services provided in accompaniment to sales of cosmetics and the like. Absolutely no attempt has been made in the past to obtain two-dimensional information in regard to the nature of oxidized protein found in the horny layer of skin.

The present invention therefore provides a method for two-dimensional evaluation of the nature of horny layer oxidized protein on a horny layer, the method being characterized by comprising the steps of specific fluorescent labeling of the carbonyl groups of oxidized protein in a horny layer specimen taken from skin, and detection of the fluorescence for evaluation. Preferably, the detection is carried out under a fluorescent microscope. More preferably, the detection results obtained from the fluorescent microscope are imaged.

According to a preferred mode, the specific fluorescent labeling of the carbonyl groups of the oxidized protein is accomplished by reacting and binding a hydrazino group-containing fluorescent substance to the oxidized protein. Preferably, the hydrazino group-containing fluorescent substance is selected from the group consisting of fluorescein-5-thiosemicarbazide and Texas red hydrazide.

According to another preferred mode, the specific fluorescent labeling of the carbonyl groups of the oxidized protein is accomplished by reacting and binding biotin hydrazide to the oxidized protein, followed by reacting and binding fluorescent-labeled avidin thereto to form a complex of the biotin hydrazide and the fluorescent-labeled avidin. Preferably, the fluorescent-labeled avidin is fluorescein-labeled avidin.

According to yet another preferred mode, the specific fluorescent labeling of the carbonyl groups of the oxidized protein is accomplished by reacting and binding dinitrophenylhydrazine to the oxidized protein, followed by reacting and binding anti-dinitrophenyl antibody thereto, and then reacting and binding a fluorescent-labeled antibody specific for the anti-dinitrophenyl antibody thereto.

Preferably, the horny layer specimen is a tape-stripped horny layer obtained by tape stripping against the skin.

The invention further provides a kit to be used in a method for two-dimensional evaluation of the presence of horny layer oxidized protein on a horny layer, the kit being characterized by comprising
adhesive tape for sampling of a horny layer specimen by tape stripping, and
a fluorescent substance for specific fluorescent labeling of the carbonyl groups of the oxidized protein. Preferably, the evaluation is carried out under a fluorescent microscope. More preferably, the detection results obtained from the fluorescent microscope are imaged.

According to a preferred mode, the fluorescent substance is a hydrazino group-containing fluorescent substance, and the specific fluorescent labeling of the carbonyl groups of the oxidized protein is accomplished by reacting and binding a hydrazino group-containing fluorescent substance to the oxidized protein. Preferably, the hydrazino group-containing fluorescent substance is selected from the group consisting of fluorescein-5-thiosemicarbazide and Texas red hydrazide.

According to another preferred mode, the fluorescent substance is fluorescent-labeled avidin, and the specific fluorescent labeling of the carbonyl groups of the oxidized protein is accomplished by reacting and binding biotin hydrazide to the oxidized protein, followed by reacting and binding fluorescent-labeled avidin thereto to form a complex of the biotin hydrazide and the fluorescent-labeled avidin. Preferably, the fluorescent-labeled avidin is fluorescein-labeled avidin.

According to yet another preferred mode, the fluorescent substance is fluorescent-labeled avidin, and the specific fluorescent labeling of the carbonyl groups of the oxidized protein is accomplished by reacting and binding dinitrophenylhydrazine to the oxidized protein, followed by reacting and binding anti-dinitrophenyl antibody thereto, and then reacting and binding a fluorescent-labeled antibody specific for the anti-dinitrophenyl antibody thereto.

Another aspect of the invention provides a screening method for agents which inhibit increase in oxidized protein. The method is characterized by treating a horny layer, for example, a horny layer specimen taken from skin, or skin itself, with an appropriate oxidizing agent and candidate agent, and then accomplishing specific fluorescent labeling of the carbonyl groups of the oxidized protein on the horny layer and detecting the fluorescence to evaluate the activity of the agent for inhibition of oxidized protein increase.

According to a preferred mode, the specific fluorescent labeling of the carbonyl groups of the oxidized protein is accomplished by reacting and binding a hydrazino group-containing fluorescent substance to the oxidized protein. Preferably, the hydrazino group-containing fluorescent substance is selected from the group consisting of fluorescein-5-thiosemicarbazide and Texas red hydrazide.

According to another preferred mode, the specific fluorescent labeling of the carbonyl groups of the oxidized protein is accomplished by reacting and binding biotin hydrazide to the oxidized protein, followed by reacting and binding fluorescent-labeled avidin thereto to form a complex of the biotin hydrazide and the fluorescent-labeled avidin. Preferably, the fluorescent-labeled avidin is fluorescein-labeled avidin.

According to yet another preferred mode, the specific fluorescent labeling of the carbonyl groups of the oxidized protein is accomplished by reacting and binding dinitrophenylhydrazine to the oxidized protein, followed by reacting and binding anti-dinitrophenyl antibody thereto, and then reacting and binding a fluorescent-labeled antibody specific for the anti-dinitrophenyl antibody thereto.

Preferably, the horny layer specimen is a tape-stripped horny layer obtained by tape stripping against the skin.

The invention further provides a method for detecting the oxidized form of the cornified envelope consisting of the water-insoluble substances in a skin-derived horny layer specimen, the method being characterized by specific fluorescent labeling of the carbonyl groups in a cornified envelope in an oxidized form and detection of the fluorescence thereof for evaluation.

According to a preferred mode, the specific fluorescent labeling of the carbonyl groups in the cornified envelope in an oxidized form is accomplished by reacting and binding a hydrazino group-containing fluorescent substance on the cornified envelope in an oxidized form. Preferably, the hydrazino group-containing fluorescent substance is selected from the group consisting of fluorescein-5-thiosemicarbazide and Texas red hydrazide.

According to another preferred mode, the specific fluorescent labeling of the carbonyl groups in the cornified envelope in oxidized form is accomplished by reacting and binding biotin hydrazide to the cornified envelope in oxidized form, followed by reacting and binding fluorescent-labeled avidin thereto to form a complex of the biotin hydrazide and the fluorescent-labeled avidin. Preferably, the fluorescent-labeled avidin is fluorescein-labeled avidin.

According to yet another preferred mode, the specific fluorescent labeling of the carbonyl groups in the cornified envelope in oxidized form is accomplished by reacting and binding dinitrophenylhydrazine to the cornified envelope in oxidized form, followed by reacting and binding of anti-dinitrophenyl antibody thereto, and then reacting and binding a fluorescent-labeled antibody specific for the anti-dinitrophenyl antibody thereto.

Even more preferably, the aforementioned method includes detection by staining of the hydrophobic regions of the cornified envelope with a dye capable of selective staining, and/or detection of the antigenicity of the cornified envelope. Preferably, the dye is Nile Red and the antigenicity is detected using anti-human involucrin antibody.

Even more preferably, the detection is carried out using a fluorescent microscope.

### Brief Description of the Drawings

Fig. 1 shows detection results for horny layer oxidized protein using biotin hydrazide.
Fig. 2 shows detection results for horny layer oxidized protein using Texas red hydrazide.
Fig. 3 shows detection results for horny layer oxidized protein using dinitrophenylhydrazine (DNPH).
Fig. 4 shows evaluation results for acetone-treated horny layer oxidized protein.
Fig. 5 shows site-specific detection results for horny layer oxidized protein using fluorescein-5-thiosemicarbazide.
Fig. 6 shows comparative results for oxidized protein in the outer layer and inner layer of the horny layer.
Fig. 7 shows results for ultraviolet irradiation- induced horny layer oxidized protein increase.
Fig. 8 shows results for ultraviolet irradiation- induced horny layer oxidized protein increase.
Fig. 9 shows results for sodium hypochlorite- and unsaturated fatty acid-induced horny layer oxidized protein increase.
Fig. 10 shows results for sodium hypochlorite-induced horny layer oxidized protein increase.
Fig. 11 shows results for unsaturated fatty acid-induced horny layer oxidized protein increase.
Fig. 12 shows results for aldehyde-induced horny layer oxidized protein increase.
Fig. 13 shows detection results for oxidized cornified envelope using biotin hydrazide.
Fig. 14 shows simultaneous comparative results for cornified envelope oxidation degree and maturation as detected utilizing anti-involucrin antibody.
Fig. 15 shows simultaneous comparative results for cornified envelope oxidation degree and maturation as detected utilizing Nile Red.

### Best Mode for Carrying Out the Invention

The invention provides a method for two-dimensional evaluation of the nature of horny layer oxidized protein on a horny layer, which method is characterized by comprising the steps of specific fluorescent labeling of the carbonyl groups of oxidized protein in a horny layer specimen taken from skin, and detection of the fluorescence for evaluation, as well as a kit used to carry out the method. The method of the invention allows specific fluorescent labeling of oxidized protein, as demonstrated by the examples which follow.

The horny layer is composed of keratinocytes developed by terminal differentiation of epidermal cornified cells, and the intracellular lipids surrounding them. Keratinocytes comprise a major component keratin as the structural protein, and are surrounded by a cornified envelope. Carbonyl groups become introduced into the horny layer protein as a result of oxidation occurring with exposure and aging, due to several factors including ultraviolet irradiation, chemical oxidation and atmospheric pollutants. The oxidation includes direct oxidation of NH₂ groups of the amino acid residues Lys, Arg and Pro in proteins to carbonyl groups, and oxidation of lipids to lipid peroxides and their further degradation to highly reactive aldehydes which bind to proteins.

According to the invention, the skin-derived horny layer specimen may be derived from any part of the body, or it may be a culture of the specimen (tissue or cells). As typical sites or regions of the body from which such specimens are derived there may be mentioned the cheeks, forehead, back of the hand or the body trunk.

The specimen may be obtained by an invasive method such as surgical means, but for the particular purpose of evaluating skin quality, it is preferably obtained from skin by a non-invasive method for convenience. As non-invasive methods there may be mentioned tape stripping and excoriation which are commonly employed in the

### technical field.

Tape stripping allows the two-dimensional condition of skin to be transferred directly to adhesive tape by attaching a side of the adhesive tape onto the skin surface and peeling it off, and it is therefore particularly preferred for the invention. By obtaining a tape horny layer by tape stripping and directly carrying out specific fluorescent staining of the oxidized protein without cutting, it is possible to obtain two-dimensional information on the oxidized protein corresponding to the actual two-dimensional condition of the skin.

A preferred method of tape stripping involves first cleaning the surface layer of the skin with ethanol or the like to remove the sebum, dirt, etc., and then lightly placing one side of adhesive tape cut to an appropriate size (for example, 5 x 5 cm) onto the skin surface, applying a uniform pressure onto the entire tape to press it down flatly, and then peeling off the adhesive tape with a uniform force. The adhesive tape used may be a commercially available cellophane tape, such as Scotch Superstrength Mailing Tape (by 3M Corp.)

The fluorescent substance for specific fluorescent labeling of the carbonyl groups of the oxidized protein to be used for the invention is preferably one having a hydrazino group:

-NHNH₂

which can bind to carbonyl groups in oxidized protein. Examples of such fluorescent substances include fluorescein-5-thiosemicarbazide, Texas red hydrazide and lucifer yellow hydrazide.

When such hydrazino group-containing fluorescent substances are used, detection of the oxidized protein can be accomplished in the following manner, for example.
(1) A horny layer specimen is obtained by tape stripping, for example;
(2) The hydrazino group-containing fluorescent substance in an appropriate buffer (for example, 100 mM MES-Na buffer (pH 5.5)) is reacted therewith at room temperature for several hours (for example, 1 hour);
(3) After completion of the reaction, thorough washing is carried out with an appropriate physiological solution (for example, phosphate-buffered saline (PBS)) and then the oxidized protein is detected with a fluorescent microscope;
(4) Optionally, a fluorescent microscope image is taken.

The specific fluorescent labeling of the oxidized protein may be performed with a combination of biotin hydrazide and fluorescent-labeled avidin. Since biotin hydrazide also contains a hydrazino group, it can bind to protein carbonyl groups. In this case, the biotin hydrazide is allowed to bind with the oxidized protein first, and then the fluorescent-labeled avidin is allowed to bind to the biotin hydrazide via biotin-avidin bonds, resulting in fluorescent labeling of the oxidized protein. Biotin hydrazide is well known in the art, and for example, the commercially available product of Pearce Co. may be used. The fluorescent avidin used may be, for example, fluorescein avidin.

When such a hydrazino group-containing fluorescent substance is used, detection of the oxidized protein may also be accomplished in the following manner, for example.
(1) A horny layer specimen is obtained by tape stripping, for example;
(2) Biotin hydrazide in an appropriate buffer (for example, 100 mM MES-Na buffer (pH 5.5)) is reacted therewith at room temperature for several hours (for example, 1 hour);
(3) After completion of the reaction, thorough washing is carried out with an appropriate physiological solution (for example, phosphate-buffered saline (PBS)), and then fluorescent-labeled avidin is reacted therewith at room temperature for several hours (for example, 1 hour);
(4) The oxidized protein is detected with a fluorescent microscope;
(5) Optionally, a fluorescent microscope image is taken.

The specific fluorescent labeling of the oxidized protein may alternatively be accomplished by allowing dinitrophenylhydrazine to react and bind to the carbonyl groups of the oxidized protein, and labeling the dinitrophenyl portion with a fluorescent dye. Thus, according to another preferred mode of the invention, detection may be accomplished by immunofluorescent measurement of the dinitrophenyl portion of dinitrophenylhydrazine bound to the carbonyl groups of oxidized protein.

When dinitrophenylhydrazine is utilized for the fluorescent labeling, detection of the oxidized protein may be accomplished in the following manner, for example.
(1) A horny layer specimen is obtained by tape stripping, for example;
(2) Dinitrophenylhydrazine (DNPH) in an appropriate buffer (for example, 100 mM MES-Na buffer (pH 5.5)) is reacted therewith at room temperature for several hours (for example, 1 hour);
(3) After completion of the reaction, thorough washing is carried out with an appropriate physiological solution (for example, phosphate-buffered saline (PBS)), and then the same physiological solution of anti-DNP antibody, for example, rabbit anti-DNP antibody (ZYMED Co.) is reacted therewith at room temperature for several hours (for example, 1 hour);
(4) After completion of the reaction, thorough washing is carried out with the same physiological solution, and then a fluorescent-labeled secondary antibody specific for the anti-DNP antibody, such as fluorescein-labeled anti-rabbit Ig (Amersham-Pharmacia Biotech), is reacted therewith at room temperature for several hours (for example, 1 hour);
(5) The oxidized protein is detected with a fluorescent microscope;
(6) Optionally, a fluorescent microscope image is taken.

A kit according to the invention may contain the aforementioned adhesive tape and fluorescent substance, together with reagents, such as various buffer solutions, necessary for carrying out the evaluation methods described above.

By using the oxidized protein detection method and kit described above, it is possible to obtain a variety of information regarding the two-dimensional condition of oxidized protein on the skin, and specifically its amount, location and distribution, such as whether it is dispersed or localized, and this can be accomplished with a fluorescent microscope as the only expensive device, without requiring protein extraction, electrophoresis, Western blotting or the like as has been necessary in the prior art. Thus, the oxidized protein detection method and kit described above can yield information which is important for evaluating skin quality, with a simple procedure and simple equipment, and they are suitable for cosmetic vendor storefronts, for example.

The present invention also provides a screening method for agents that inhibit oxidized protein increase. The method is characterized by treating a horny layer, for example, a horny layer specimen taken from skin, or skin itself, with an appropriate oxidizing agent and candidate agent, and then accomplishing specific fluorescent labeling of the carbonyl groups of the oxidized protein on the horny layer and detecting the fluorescence to evaluate the activity of the agent for inhibition of oxidized protein increase. The oxidizing agent used may be any of various oxidizing agents which are well known to those skilled in the art, such as sodium hypochlorite, unsaturated fatty acids including linoleic acid, oleic acid and palmitoleic acid, and aldehydes such as acrolein. Treatment of the horny layer specimen with the oxidizing agent may be carried out at the same time as treatment with the candidate agent, or it may be carried out after treatment with the candidate agent. According to a preferred mode, treatments of the horny layer specimen with the oxidizing agent and the candidate agent are carried out simultaneously.

Specifically, the screening method for agents that inhibit oxidized protein increase may be carried out in the following manner, for example.

Adhesive tape is attached to the skin, such as an unexposed part of the abdomen, of a healthy individual, and immediately stripped off for non-invasive sampling of the outer layer of the horny layer. The tape which was attached to the horny layer is incubated in an aqueous solution containing a mixture of the oxidizing agent and a fixed concentration of the candidate agent. Upon thorough washing after incubation is complete, the carbonyl groups of the oxidized protein are subjected to specific fluorescent labeling as described above, and after further washing, a fluorescent microscope or the like is used for observation to evaluate to what extent the candidate agent has inhibited increase in oxidized protein due to the oxidizing agent. For the evaluation, selection may be made based on the concentration of the agent which inhibits increase of oxidized protein down to the median value between the value after incubation with the oxidizing agent alone and the value after incubation with water alone, and for example, an agent which inhibits increase in oxidized protein can be prepared with a concentration of no greater than 1 mM, preferably no greater than 500 µM and more preferably no greater than 250 µM. The inhibiting effect of the agent on oxidized protein increase may also be evaluated based on the level of oxidized protein in the horny layer specimen obtained after simultaneously applying the oxidizing agent onto human skin.

The invention further provides a method for detecting the oxidized form of the cornified envelope consisting of the water-insoluble substances in a skin-derived horny layer specimen, which method is characterized by comprising the steps of specific fluorescent labeling of the carbonyl groups in a cornified envelope in oxidized form, and specifically the oxidized protein in a cornified envelope in oxidized form, and detection of the fluorescence thereof for evaluation.

As explained above, keratinocytes comprise keratin fibers as a major component, with a cornified envelope surrounding them. The cornified envelope is formed when a plurality of cornified envelope precursor proteins, which are produced as epidermal keratinocytes differentiate, become crosslinked and insolubilized by the enzyme transglutaminase. Presumably, ceramides and other components covalently bond to a portion thereof to form a hydrophobic structure, serving as a foundation for the aforementioned intracellular lamellar structure and forming the basis of the barrier function of the horny layer. The cornified envelope can be prepared by boiling skin tissue or cultured skin cells in a solution containing a surfactant such as sodium dodecylsulfate and a reducing agent such as mercaptoethanol, and obtaining the insoluble fraction having the soluble components removed by means such as centrifugal separation. Morphological examination under a microscope allows the condition thereof to be evaluated. Michel et al. have reported that a fragile cornified envelope is more abundant in the deeper sections of the horny layer than in the outer layer of the horny layer (J. Invest. Dermatol. 91:11-15, 1988). However, a fragile cornified envelope is also found in the outer layer in such conditions as psoriasis and folliated ichthyosis (Br. J. Dermatol. 122:15-21, 1990).

The relationship between lipids and the cornified envelope, which are mainly responsible for the barrier function of the horny layer, and particularly the function of preventing evaporation of moisture from the body and infiltration of foreign matter from the exterior, as well as the nature of the cornified envelope, have been the focus of attention and methods for their evaluation have been disclosed (Japanese Unexamined Patent Publication No. 2001-91514). However, the prior art includes no disclosure regarding evaluation of the oxidized form of the cornified envelope. As explained above, the cornified envelope is also composed of protein, and therefore its oxidized form can be detected in the same manner by applying the method for detecting horny layer oxidized protein according to the invention.

A specific method for evaluating the oxidized form of the cornified envelope may be carried out in the following manner, for example.
(1) A horny layer specimen is obtained by tape stripping, for example;
(2) Biotin hydrazide in an appropriate buffer (for example, 100 mM MES-Na buffer (pH 5.5)) is reacted therewith at room temperature for several hours (for example, 1 hour);
(3) After completion of the reaction, an aqueous extract (for example, a 0.1 M Tris-HCl buffer solution (pH 8.5) containing 2% sodium dodecylsulfate, 20 mM dithiothreitol and 5 mM EDTA) is added to the cornified envelope obtained as the water-insoluble fraction, the mixture is subjected to heat treatment (for example, 10 min at 100°C), and the insoluble portion is separated by centrifugation (for example, 4000 g, 10 min) and collected as the cornified envelope;
(4) The procedure of (3) is repeated to thoroughly remove the soluble components.
(5) The cornified envelope suspension is dropped onto a glass slide, air-dried and acetone-fixed, after which thorough washing is carried out with an appropriate physiological solution (for example, phosphate-buffered saline (PBS)), and then fluorescent-labeled avidin is reacted therewith at room temperature for several hours (for example, 1 hour);
(6) After completion of the reaction, thorough washing is carried out with the same physiological solution and the oxidized cornified envelope is detected with a fluorescent microscope;
(7) Optionally, a fluorescent microscope image is taken.

For evaluation of a cornified envelope in a mature form, the oxidized cornified envelope detection method may optionally be carried out by staining of the hydrophobic regions with a selectively staining dye, or for evaluation of a cornified envelope in an immature form, it may be carried out by detection of antigenicity with respect to involucrin antibody.

Dyes which may be used for selective staining of hydrophobic regions (particularly of biotissue) include those commonly used for staining of hydrophobic regions. As specific dyes there may be mentioned Nile Red, Oil Red O and Sudan III. Nile Red is particularly preferred for use. Nile Red may also be in admixture with its reduced form, Nile Blue. Such a mixture may also be in a form wherein a portion of the Nile Blue in a Nile Blue aqueous solution is naturally converted to Nile Red.

As target proteins for detection of antigenicity of the constituent proteins of the cornified envelope there may be mentioned proteins such as involucrin and loricrin whose lipids are capable of covalent bonding, or isopeptides and pseudo-isopeptides having crosslinked bonds between proteins. The antigenicity can be detected using antibody for the protein or peptide. The detection method may be any method which allows detection of antibody bonds on proteins or peptides, but the fluorescent antibody method, which is used for labeling or staining of antigenic substances such as enzymes or structural proteins in tissue samples, is preferred. Examples of fluorescent labels for the antibody include fluorescein isothiocyanate (FITC), tetramethylrhodamine isothiocyanate (TRITC) and the like.

In the method for evaluation of the nature of the cornified envelope, for example, when it is possible to significantly detect the involucrin in the cornified envelope of the horny layer specimen by an antigenicity detection method, a value up to a significant level (or to an identifiable level) means either that lipids are not covalently bonded to the involucrin, or that the crosslinking reaction is not sufficient and antigenicity is maintained, and therefore the cornified envelope of the horny layer specimen may be evaluated as immature. Conversely, for example, when the involucrin cannot be significantly detected, this means that covalent bonding of lipids to the involucrin is considerable or crosslinking reaction has sufficiently proceeded to eliminate antigenicity, and therefore the cornified envelope may be evaluated as mature. Also, for example, when a cornified envelope which is strongly dye-positive with Nile Red is detected (or observed) in the horny layer specimen, the cornified envelope may be evaluated as stiff. Conversely, when diversity is found in the dyeability with Nile Red, it may be evaluated that variation is present in the cornified envelope. Such evaluations can be applied in the same manner for the cornified envelope-formation process for epidermal cornified cells.

### Examples

The present invention will now be explained in greater detail by the following examples.

### Example 1

### Detection of horny layer oxidized protein using biotin hydrazide

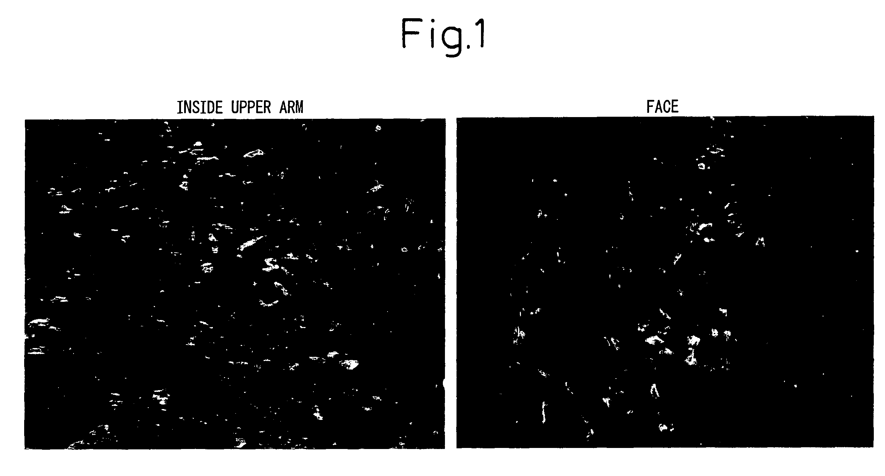

Transparent adhesive tape was attached to the face and inside upper arm of a healthy individual and immediately stripped off, to non-invasively obtain the outer layer of the horny layer. A 100 mM MES-Na buffer solution (pH 5.5) containing 5 mM biotin hydrazide (PIERCE Co.) was reacted therewith at room temperature for 1 hour. After completion of the reaction, thorough washing was carried out with phosphate-buffered saline (PBS), and then fluorescein-labeled avidin (product of Amersham-Pharmacia Biotech, diluted to 1:100 volume with PBS) was reacted therewith at room temperature for 1 hour. After completion of the reaction, thorough washing was carried out with PBS and a fluorescent microscope (Olympus BX52) was used for observation. The observed image is shown in Fig. 1.

As seen in Fig. 1, fluorescence was observed for both the inside upper arm and the face, indicating that oxidized protein had been sufficiently labeled with the biotin hydrazide-fluorescein-labeled avidin, and was detectable.

### Example 2

### Detection of horny layer oxidized protein using Texas Red hydrazide

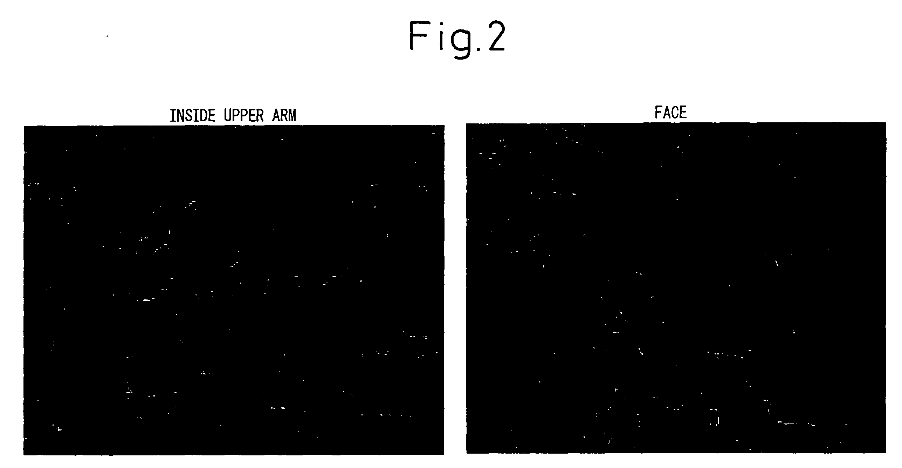

Transparent adhesive tape was attached to the face (cheek) and inside upper arm of a healthy individual and immediately stripped off, to non-invasively obtain the outer layer of the horny layer. A 100 mM MES-Na buffer solution (pH 5.5) containing 20 µM Texas red hydrazide (Molecular Probes Co.) was reacted therewith at room temperature for 1 hour. After completion of the reaction, thorough washing was carried out with phosphate-buffered saline (PBS), and then a fluorescent microscope was used for observation. The observed image is shown in Fig. 2

As seen in Fig. 2 fluorescence was observed for both the inside upper arm and the face, thus clearly demonstrating that the oxidized protein was also labeled with Texas Red hydrazide, similar to fluorescein-5-thiosemicarbazide.

### Example 3

### Detection of horny layer oxidized protein using dinitrophenylhydrazine (DNPH)

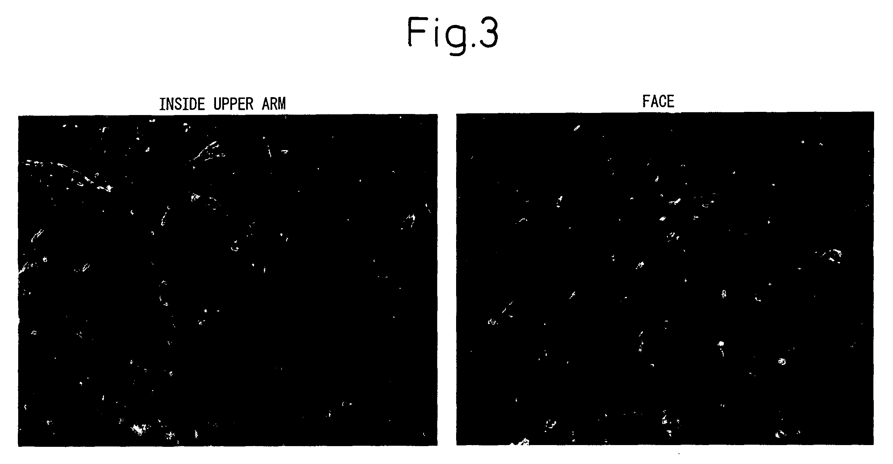

Transparent adhesive tape was attached to the face and inside upper arm of a healthy individual and immediately stripped off, to non-invasively obtain the outer layer of the horny layer. A 100 mM MES-Na buffer solution (pH 5.5) containing 200 µM dinitrophenylhydrazine (Wako Pure Chemical Industries Co., Ltd.) was reacted therewith at room temperature for 1 hour. After completion of the reaction, thorough washing was carried out with phosphate-buffered saline (PBS), and then a PBS solution (1 µg/ml) of rabbit anti-DNP antibody (ZYMED Co.) was reacted therewith at room temperature for 1 hour. After completion of the reaction, fluorescein-labeled anti-rabbit Ig (product of Amersham-Pharmacia Biotech, diluted to 1:100 volume with PBS) was reacted therewith at room temperature for 1 hour. After completion of the reaction, thorough washing was carried out with PBS and a fluorescent microscope was used for observation. The observed image is shown in Fig. 3.

As seen in Fig. 3, fluorescence was observed for both the inside upper arm and the face, demonstrating that oxidized protein can be specifically labeled using dinitrophenylhydrazine, as with biotin hydrazide, fluorescein-5-thiosemicarbazide and Texas Red hydrazide.

### Example 4

### Evaluation of acetone-treated horny layer oxidized protein

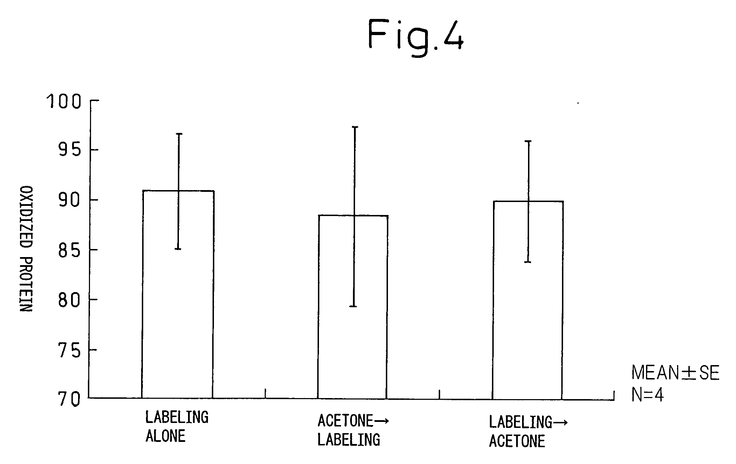

Transparent adhesive tape was attached to the faces of four healthy individuals and immediately stripped off to non-invasively obtain the outer layer of the horny layer, and three specimens were prepared for each individual. One of the specimens was treated with acetone for 30 minutes, and the other two specimens were not treated. (Here, the acetone-treated specimen was designated as the "acetone→labeling" specimen and the other two were designated as the "labeling only" or "labeling→acetone" specimen.) A 100 mM MES-Na buffer solution (pH 5.5) containing 20 µM fluorescein-5-thiosemicarbazide (Molecular Probes Co.) was reacted with each specimen at room temperature for 1 hour. After completion of the reaction, thorough washing was carried out with phosphate-buffered saline (PBS), and only the "labeling→acetone" specimen was treated with acetone for 30 minutes. Each specimen was then observed with a fluorescent microscope. The backgrounds and cells were resolved from the images, and the average brightnesses were determined for numerical quantitation of the oxidized protein. A numerical graph is shown in Fig. 4.

Fig. 4 shows that no significant change is found in signal strength even when the tape horny layer is treated with acetone. This suggested that most of the carbonyl groups labeled with fluorescent hydrazide were derived from oxidized protein, and that the proportion of lipid peroxides present in the horny layer was low.

It was thus suggested that the present invention is highly advantageous for detection of oxidized protein in the horny layer.

### Example 5

### Detection of site-specific differences in horny layer oxidized protein with fluorescein-5-thiosemicarbazide

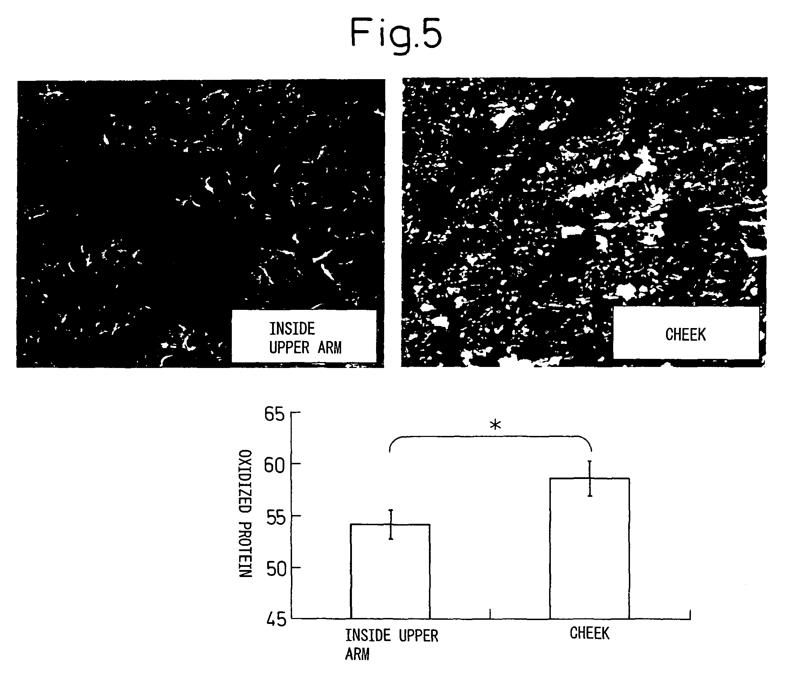

Transparent adhesive tape was attached to the face (cheek) and inside upper arm of a healthy individual and immediately stripped off, to non-invasively obtain the outer layer of the horny layer. A 100 mM MES-Na buffer solution (pH 5.5) containing 20 µM fluorescein-5-thiosemicarbazide (Molecular Probes Co.) was reacted therewith at room temperature for 1 hour. After completion of the reaction, thorough washing was carried out with phosphate-buffered saline (PBS), and then a fluorescent microscope was used for observation. For numerical quantitation, the backgrounds and cells were resolved from the images, and the average brightnesses were determined. The observed images and a numerical graph are shown in Fig. 5.

As seen in Fig. 5, fluorescence was observed for both the inside upper arm and the face, and clearly based on both the observed images and the numericalized graph, the amount of oxidized protein on the cheeks, i.e. the exposed area, was greater than on the inside upper arm, i.e. the unexposed area, thus matching the conclusion in J.J. Thiele et al. FEBS Letter 1998, Feb.6, 422(3), 403-406. Thus, it was demonstrated that oxidized protein is sufficiently labeled with biotin hydrazide-fluorescein-labeled avidin, and is detectable.

### Example 6

### Detection of oxidized protein in outer layer and inner layer of horny layer with fluorescein-5-thiosemicarbazide

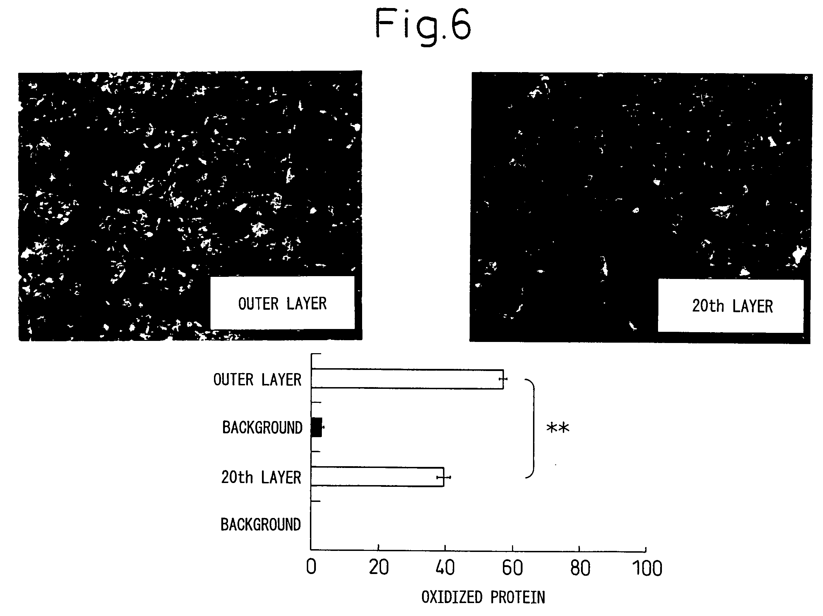

Transparent adhesive tape was attached to the inside upper arm of a healthy individual and immediately stripped off, and this was repeated 20 times to non-invasively obtain the outer layer of the horny layer and the 20th horny layer. A 100 mM MES-Na buffer solution (pH 5.5) containing 20 µM fluorescein-5-thiosemicarbazide (Molecular Probes Co.) was reacted therewith at room temperature for 1 hour. After completion of the reaction, thorough washing was carried out with phosphate-buffered saline (PBS), and then a fluorescent microscope was used for observation. For numerical quantitation, the backgrounds and cells were resolved from the images, and the average brightnesses were determined. The observed images and a numerical graph are shown in Fig. 6.

As seen in Fig. 6, based on both the observed images and the numericalized graph, the amount of oxidized protein was greater on the outer layer than in the 20th horny layer, indicating greater oxidized protein toward the upper layer of the horny layer, thus matching the observation in J.J. Thiele et al. J. Invest. Dermatol. 1999, Sep ,113(3), 335-359.

### Example 7

### Increase in horny layer oxidized protein due to ultraviolet irradiation

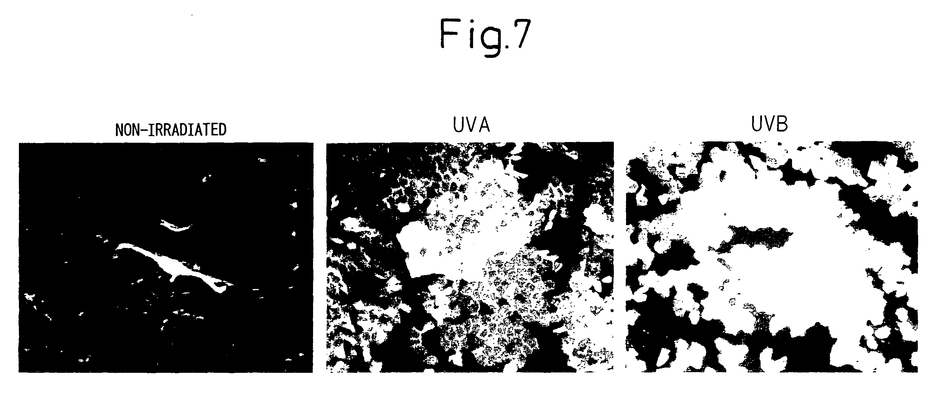

Transparent adhesive tape was attached to the inside upper arm of a healthy individual and immediately stripped off, to non-invasively obtain the outer layer of the horny layer. The tape attached to the horny layer was irradiated with UVA (240 J/cm²) and UVB (77 J/cm²), and then a 100 mM MES-Na buffer solution (pH 5.5) containing 20 µM fluorescein-5-thiosemicarbazide (Molecular Probes Co.) was reacted therewith at room temperature for 1 hour. After completion of the reaction, thorough washing was carried out with phosphate-buffered saline (PBS), and then a fluorescent microscope was used for observation. The observed images are shown in Fig. 7. An increase in oxidized protein was seen with ultraviolet irradiation, in agreement with C.S. Sander et al. J. Invest. Dermatol. 2002, Apr, 118(4), 618-625.

### Example 8

### Increase in horny layer oxidized protein due to ultraviolet irradiation

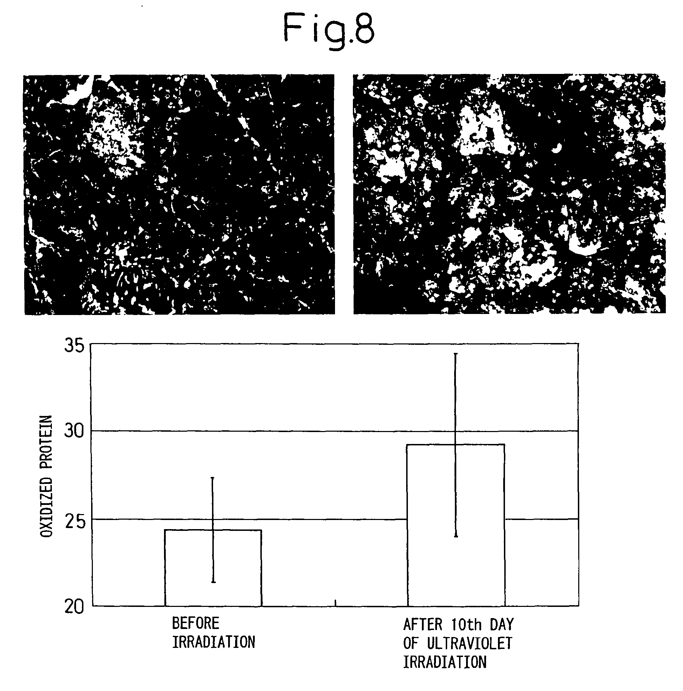

On the 10th day after 2 MED UVB irradiation of the back of a healthy individual, transparent adhesive tape was attached to the irradiated site and immediately stripped off, to non-invasively obtain the outer layer of the horny layer. The tape attached to the horny layer was reacted with a 100 mM MES-Na buffer solution (pH 5.5) containing 20 µM fluorescein-5-thiosemicarbazide (Molecular Probes Co.) at room temperature for 1 hour. After completion of the reaction, thorough washing was carried out with phosphate-buffered saline (PBS), and then a fluorescent microscope was used for observation. The observed images and a numerical graph are shown in Fig. 8. An increase in oxidized protein was seen with ultraviolet irradiation, compared to before irradiation.

### Example 9

### Increase in horny layer oxidized protein due to sodium hypochlorite and unsaturated fatty acids

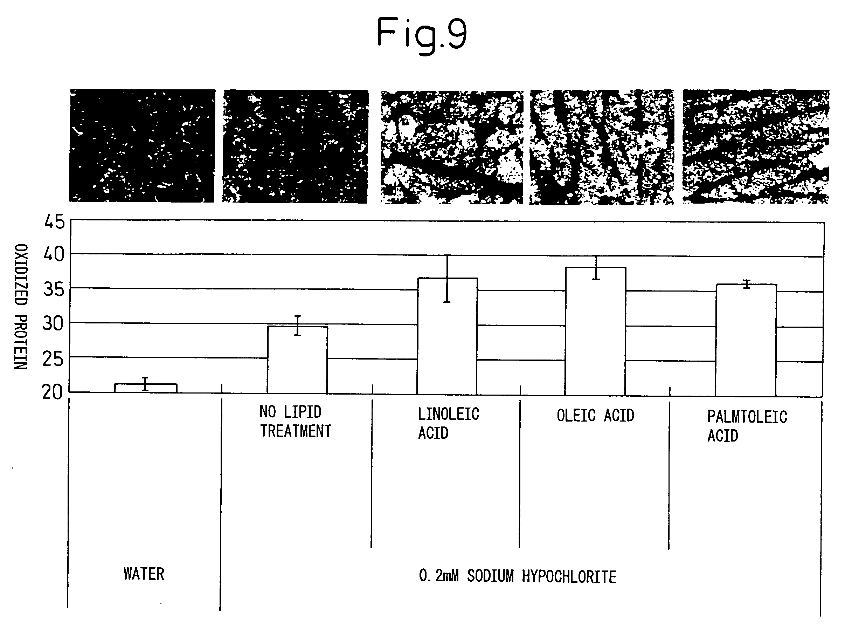

Transparent adhesive tape was attached to the abdomen of a healthy individual and immediately stripped off, to non-invasively obtain the outer layer of the horny layer. The tape attached to the horny layer was coated with 200 µg/cm² of unsaturated fatty acids (linoleic acid, oleic acid and palmitoleic acid), and then incubated in a 0.2 mM aqueous sodium hypochlorite solution at 37°C for 18 hours. After incubation was complete, the mixture was thoroughly washed with phosphate-buffered saline (PBS) and then reacted with a 100 mM MES-Na buffer solution (pH 5.5) containing 20 µM fluorescein-5-thiosemicarbazide (Molecular Probes Co.) at room temperature for 1 hour. After completion of the reaction, thorough washing was carried out with phosphate-buffered saline (PBS) and a fluorescent microscope was used for observation. The observed images and a numerical graph are shown in Fig. 9. An increase in oxidized protein was found after incubation with sodium hypochlorite, compared to incubation with water. Also, a further increase in oxidized protein was found after coating of the horny layer adhesive tape with unsaturated fatty acids and incubation with sodium hypochlorite.

### Example 10

### Increase in horny layer oxidized protein due to sodium hypochlorite

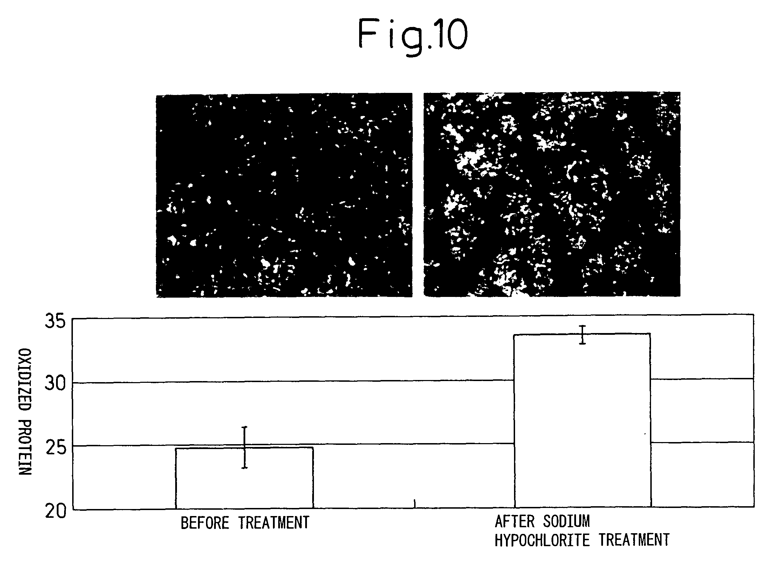

After coating the forearm of a healthy individual with 2 µL/cm² of a 20 mM aqueous sodium hypochlorite solution for 5 consecutive days, transparent adhesive tape was attached and immediately stripped off, to non-invasively obtain the outer layer of the horny layer. The tape attached to the horny layer was reacted with a 100 mM MES-Na buffer solution (pH 5.5) containing 20 µM fluorescein-5-thiosemicarbazide (Molecular Probes Co.) at room temperature for 1 hour. After completion of the reaction, thorough washing was carried out with phosphate-buffered saline (PBS), and then a fluorescent microscope was used for observation. The observed images and a numerical graph are shown in Fig. 10. An increase in oxidized protein was seen as a result of coating with sodium hypochlorite for the consecutive day period.

### Example 11

### Increase in horny layer oxidized protein due to unsaturated fatty acid

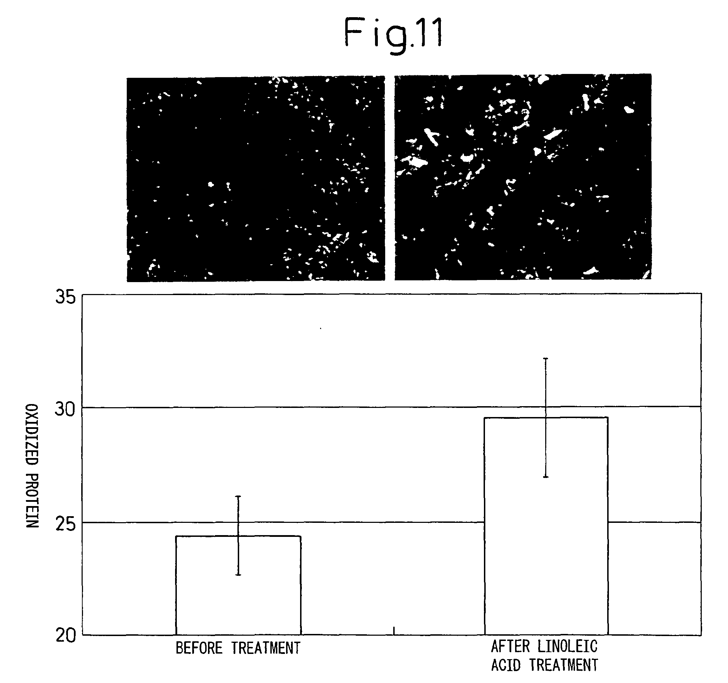

After coating the forearm of a healthy individual with 40 µL/cm² of a 30% linoleic acid-ethanol solution for 3 consecutive days, transparent adhesive tape was attached to the irradiated site and immediately stripped off, to non-invasively obtain the outer layer of the horny layer. The tape attached to the horny layer was reacted with a 100 mM MES-Na buffer solution (pH 5.5) containing 20 µM fluorescein-5-thiosemicarbazide (Molecular Probes Co.) at room temperature for 1 hour. After completion of the reaction, thorough washing was carried out with phosphate-buffered saline (PBS), and then a fluorescent microscope was used for observation. The observed images and a numerical graph are shown in Fig. 11. An increase in oxidized protein was seen as a result of coating with linoleic acid for the consecutive day period.

### Example 12

### Increase in horny layer oxidized protein due to aldehyde

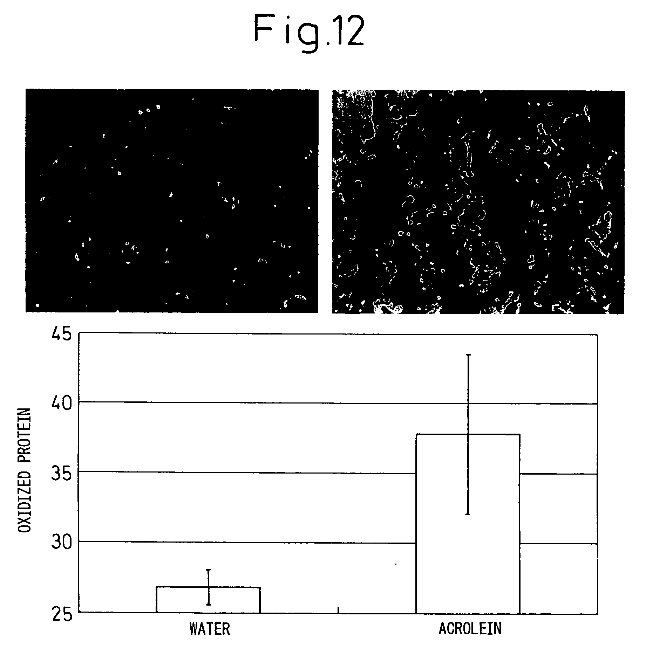

Transparent adhesive tape was attached to the abdomen of a healthy individual and immediately stripped off, to non-invasively obtain the outer layer of the horny layer. The tape attached to the horny layer was incubated in a 1 mM aqueous acrolein solution at room temperature for 18 hours. After incubation was complete, the mixture was thoroughly washed with phosphate-buffered saline (PBS) and then reacted with a 100 mM MES-Na buffer solution (pH 5.5) containing 20 µM fluorescein-5-thiosemicarbazide (Molecular Probes Co.) at room temperature for 1 hour. After completion of the reaction, thorough washing was carried out with phosphate-buffered saline (PBS) and a fluorescent microscope was used for observation. The observed images and a numerical graph are shown in Fig. 12. An acrolein- induced increase in oxidized protein was found.

### Example 13

### Agent-induced inhibition of increase in horny layer oxidized protein

Transparent adhesive tape was attached to the abdomen of a healthy individual and immediately stripped off, to non-invasively obtain the outer layer of the horny layer. The tape attached to the horny layer was incubated in an aqueous solution comprising a mixture of 0.2 mM sodium hypochlorite and a fixed concentration of the agent, at 37°C for 18 hours. After incubation was complete, the mixture was thoroughly washed with phosphate-buffered saline (PBS) and then reacted with a 100 mM MES-Na buffer solution (pH 5.5) containing 20 µM fluorescein-5-thiosemicarbazide (Molecular Probes Co.) at room temperature for 1 hour. After completion of the reaction, thorough washing was carried out with phosphate-buffered saline (PBS) and a fluorescent microscope was used for observation. The agent was demonstrated to inhibit sodium hypochlorite-induced increase in oxidized protein. Table 1 shows different agent concentrations at which oxidized protein increase was inhibited to the median value between the value obtained by incubation with sodium hypochlorite alone and the value obtained by incubation with water alone.

**Table 1**

| | 50% inhibition concentration (µM) |
|---|---|
| Ascorbic acid | 210 |
| α-Tocopherol | 220 |
| 2-O-α-D-Glucopyranosyl-L-ascorbic acid | 190 |
| 3-O-Ethylascorbic acid | 140 |
| 2-Aminoethanesulfinic acid | 86 |
| Glutathione | 8 |
| 2-Aminoethanethiosulfonic acid | 63 |
| Potassium α-tocopherol-2-L-ascorbate phosphate diester | <125 |
| Magnesium ascorbate phosphate ester | <125 |
| Sodium pyrosulfite | <125 |

### Example 14

### Detection of oxidized cornified envelope using biotin hydrazide

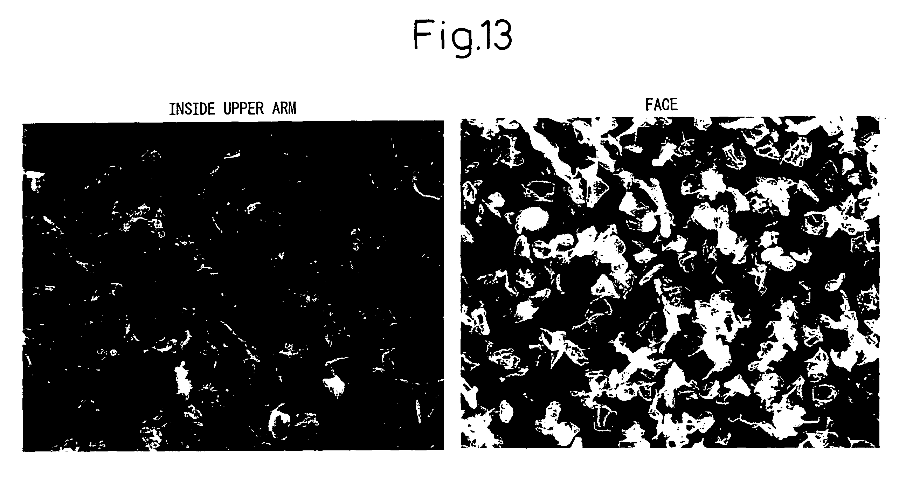

Transparent adhesive tape was attached to the face and inside upper arm of a healthy individual and immediately stripped off, to non-invasively obtain the outer layer of the horny layer. A 100 mM MES-Na buffer solution (pH 5.5) containing 5 mM biotin hydrazide (PIERCE Co.) was reacted therewith at room temperature for 1 hour. After completion of the reaction, a 0.1 M Tris-HCl buffer (pH 8.5) containing 2% sodium dodecylsulfate, 20 mM dithiothreitol and 5 mM EDTA was added and the mixture was heated at 100°C for 10 minutes. The insoluble portion was collected by centrifugal separation at 4000 g, 10 min. Eluent addition and heating were repeated to thoroughly remove the soluble components. The insoluble portion obtained by this procedure was used as the cornified envelope. The cornified envelope suspension was dropped onto a glass slide, air dried and acetone-fixed, thorough washing was carried out with phosphate-buffered saline (PBS), and then fluorescein-labeled avidin (product of Amersham-Pharmacia Biotech, diluted to 1:100 volume with PBS) was reacted therewith at room temperature for 1 hour. After completion of the reaction, thorough washing was carried out with PBS and a fluorescent microscope was used for observation. The observed image is shown in Fig. 13.

Fig. 13 shows detection of the cornified envelope in oxidized form (oxidized cornified envelope), wherein a greater amount was detected in the horny layer of the exposed face area than in the unexposed inside upper arm area.

### Example 15

### Simultaneous comparison of cornified envelope oxidation degree and maturation

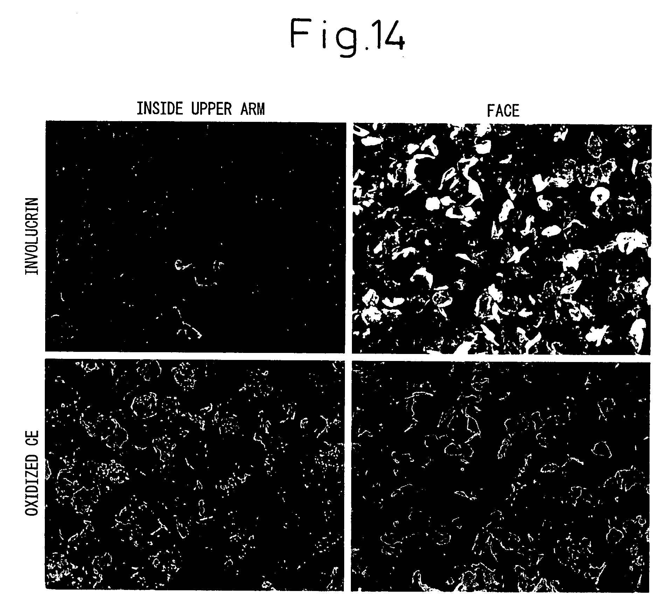

Transparent adhesive tape was attached to the face and inside upper arm of a healthy individual and immediately stripped off, to non-invasively obtain the outer layer of the horny layer. A 100 mM MES-Na buffer solution (pH 5.5) containing 200 µM dinitrophenylhydrazine (Wako Pure Chemical Industries Co., Ltd.) was reacted therewith at room temperature for 1 hour. After completion of the reaction, a 0.1 M Tris-HCl buffer (pH 8.5) containing 2% sodium dodecylsulfate, 20 mM dithiothreitol and 5 mM EDTA was added and the mixture was heated at 100°C for 10 minutes. The insoluble portion was collected by centrifugal separation at 4000 g, 10 min. Eluent addition and heating were repeated to thoroughly remove the soluble components. The insoluble portion obtained by this procedure was used as the cornified envelope. The cornified envelope suspension was dropped onto a glass slide, air dried and acetone-fixed, thorough washing was carried out with phosphate-buffered saline (PBS), and then rabbit anti-DNP antibody (ZYMED Co., 1 µg/ml) was reacted therewith for detection of the DNP-labeled oxidized cornified envelope, while simultaneously mouse anti-human involucrin antibody (NOVOCASTRA Co.) was reacted therewith as primary antibody for detection of the immature cornified envelope. After removing the excess antibody by washing, Texas Red-labeled anti-rabbit immunoglobulin and FITC-labeled anti-mouse immunoglobulin (both products of Amersham-Pharmacia Biotech, diluted to 1:100 volume with PBS) were reacted therewith as secondary antibodies at room temperature for 1 hour. After completion of the reaction, thorough washing was carried out with PBS and a fluorescent microscope was used for observation. The observed image is shown in Fig. 14.

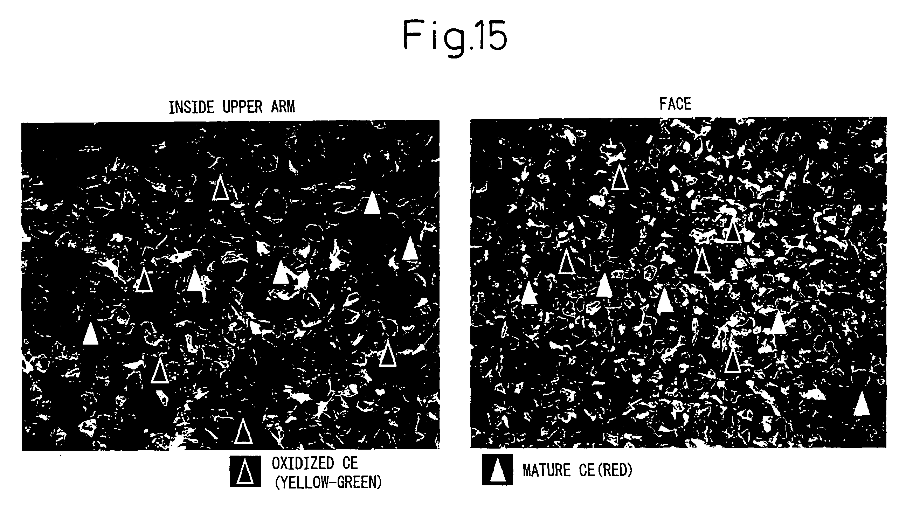

In the same manner, DNP-labeled oxidized cornified envelope was detected using rabbit anti-DNP antibody and FITC-labeled anti-mouse immunoglobulin while the mature cornified envelope was stained with Nile Red dye, and a fluorescent microscope was used for observation. The observed image is shown in Fig. 15. In this image, the mature cornified envelope stained red with Nile Red is indicated by open triangles, and the cornified envelope in oxidized form (oxidized cornified envelope) stained yellow-green with FITC is indicated by closed triangles.

Fig. 15 shows that oxidized cornified envelope was present in a greater amount in the horny layer of the exposed face area than in the unexposed inside upper arm area.

### Industrial Applicability

According to the present invention, it is possible to easily obtain two-dimensional information on the nature of oxidized protein of the skin horny layer on the skin, and such information on oxidized protein is expected to be useful particularly for counseling services provided in accompaniment to sales of cosmetics and the like.

## Claims

1. A method for two-dimensional evaluation of the nature of horny layer oxidized protein on a horny layer, the method being **characterized by** comprising the steps of specific fluorescent labeling of the carbonyl groups of said oxidized protein in a horny layer specimen taken from skin, and detection of the fluorescence for evaluation.

2. A method according to claim 1, wherein said detection is carried out under a fluorescent microscope, and the detection results obtained therefrom are imaged.

3. A method according to claim 1 or 2, wherein the specific fluorescent labeling of the carbonyl groups of said oxidized protein is accomplished by reacting and binding a hydrazino group-containing fluorescent substance to said oxidized protein.

4. A method according to any one of claims 1 to 3, wherein said horny layer specimen is a tape horny layer obtained by tape stripping against the skin.

5. A kit to be used in a method for two-dimensional evaluation of the presence of horny layer oxidized protein on a horny layer, the kit being **characterized by** comprising
adhesive tape for sampling of a horny layer specimen by tape stripping, and
a fluorescent substance for specific fluorescent labeling of the carbonyl groups of the oxidized protein.

6. A kit according to claim 5, wherein said evaluation is carried out under a fluorescent microscope, and the detection results obtained therefrom are imaged.

7. A kit according to claim 5 or 6, wherein said fluorescent substance is a hydrazino group-containing fluorescent substance, and the specific fluorescent labeling of the carbonyl groups of said oxidized protein is accomplished by reacting and binding a hydrazino group-containing fluorescent substance to said oxidized protein.

8. A screening method for agents which inhibit oxidized protein increase, the method being **characterized by** comprising the steps of treating a horny layer with an appropriate oxidizing agent and candidate agent, and then accomplishing specific fluorescent labeling of the carbonyl groups of the oxidized protein on the horny layer and detecting the fluorescence to evaluate the activity of said agent for inhibiting oxidized protein increase.

9. A method according to claim 8, wherein the specific fluorescent labeling of the carbonyl groups of said oxidized protein is accomplished by reacting and binding a hydrazino group-containing fluorescent substance to said oxidized protein.

10. A method for detecting the oxidized form of a cornified envelope consisting of the water-insoluble substances in a skin-derived horny layer specimen, the method being **characterized by** specific fluorescent labeling of the carbonyl groups in a cornified envelope in said oxidized form and detection of the fluorescence thereof for evaluation.

11. A method according to claim 10, wherein the specific fluorescent labeling of the carbonyl groups of the cornified envelope in said oxidized form is accomplished by reacting and binding a hydrazino group-containing fluorescent substance to the cornified envelope in said oxidized form.

12. A method according to claim 10 or 11, which further comprises detection by staining of the hydrophobic regions of said cornified envelope with a dye capable of selective staining, and/or detection of the antigenicity of said cornified envelope.

13. A method according to claim 12, wherein said antigenicity is detected using anti-human involucrin antibody.

14. A method according to any one of claims 9 to 13, wherein said detection is accomplished using a fluorescent microscope.
